# EUROPEAN PATENT APPLICATION

(11) **EP 1 669 025 A1**
(43) Date of publication of application: **14.06.2006**
(21) Application number: 05257408.4
(22) Date of filing: 01.12.2005
(51) Int. Cl.: A61B 5/0408, A41D 13/12, A61N 1/04

(54) **Garment electrode connection system**

(30) Priority: 07.12.2004 DE 102004058819
(71) Applicant: Dräger Safety AG & Co KGaA, 23560 Lübeck (DE)
(72) Inventor: Sattler, Frank, 23560 Lübeck (DE); Sliepen, Robert, 52062 Aachen (DE)
(74) Representative: Greenwood, John David

(57) **Abstract**

An arrangement for the transmission of an electrical signal detected by contact with the skin surface to an analysing unit including a stack of at least two clothing layers (3, 7) with an electrical line connection (4, 8) through each of the clothing layers (3, 7); in which the undermost clothing layer (3) is provided with an electrode contactable with the skin surface of the body (1) of a wearer (2) of the clothing and with an electrically conductive surface (5) on the side facing away from the skin surface, in use; and further clothing layer (7) stacked upon the undermost clothing layer (3) is provided on the underside and upper side with an electrically conductive surface (6, 9); and in which a carrier belt (11) passed over the stack of clothing layers (3, 7) is provided with an electrically conductive surface (10) facing the uppermost clothing layer (7), said conductive surface being connected by means of an electrical line (12) to the analysing unit (14).

## Description

The invention relates to an arrangement for the transmission of an electrical signal detected by contact with the skin surface to an analysing unit.

It is known to monitor persons who have to work in physically very stressful situations with regard to physiological parameters relevant to health. These include, for example, the heart rate (HR) or voltage potentials measured at the skin surface by means of electrodes, said voltage potentials being analysed for an ECG and thus revealing the current personal state of health of the monitored person in terms of heart and circulation, i.e. the condition of a firefighter deployed at a fire for example. In the event that the current physical condition appears absolutely life-threatening, either an acoustic and/or optically perceptible alarm is triggered or the operational control room is at least informed so that appropriate measures can be taken.

A non-detachable connection by means of cables is known for making the electrical contact of the individual electrodes with the respective ECG analysing electronics. A prerequisite here is that the electrodes are previously placed manually on the skin surface. In the case of a detachable connection by means of plug-in connections, the cables also have to be routed securely, and the provision of plug-in connections also requires additional work steps. Moreover, in the fire service sector, for example, particularly high demands in terms of safety and resistance to fire, high temperatures, aggressive gases and other things have to be met at the plug-in connections.

There is known from GB 2 350 193 A an arrangement for producing an electrical connection between two textiles, so that an electrical signal, for an ECG for example, is transmitted from one side of a material, which lies adjacent to the body surface, to the other side and is further processed there.

The present invention seeks to provide an arrangement for the transmission of an electrical signal detected by contact with the skin surface to an analysing unit with an improved electrical connection technique, which manages without additional work steps for the connection and without plugs.

The present invention is as claimed in the claims.

By putting on several articles of clothing and by means of the several layers of clothing thus formed and the subsequent passing of a carrier belt over the stack of at least two layers of clothing, an electrical connection between the electrode of the undermost layer of clothing making contact with the skin surface of the wearer of the clothing and the analysing unit for the signals of the electrode is produced in a rapid and straightforward manner with the specified arrangement.

Optional features of the invention are recited in the dependent claims.

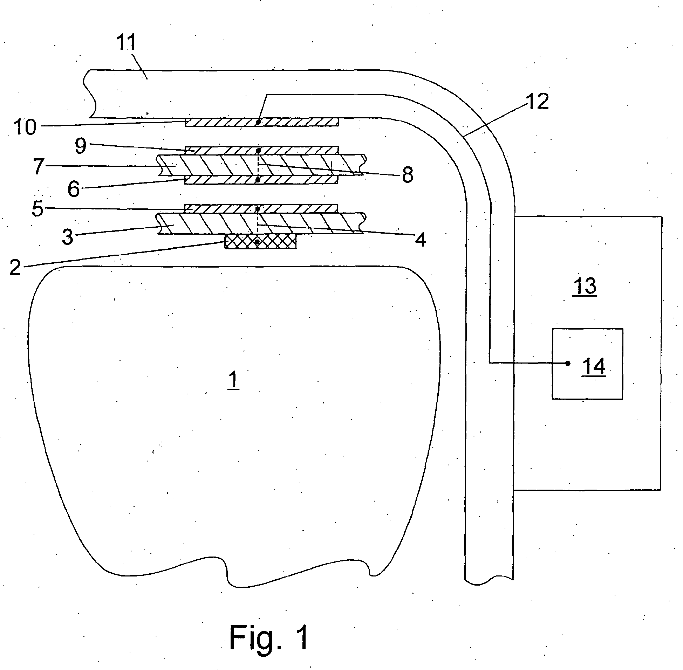

Examples of embodiments of the present invention are explained below with the aid of the diagrammatic single figure, Figure 1, of a reference electrode with several articles of clothing with assigned clothing layers and a carrier belt for respiratory equipment.

Clothing layers 3, 7 shown diagrammatically and, optionally, further layers of clothing which are assigned to corresponding articles of clothing are each provided with an electrical line connection 4, 8 between upper side and underside. Electrical line connection 4, 8 through clothing layers 3, 7 and, optionally, further clothing layers is brought about in particular by electrically conductive threads, plug-in elements or push-contact elements, which make contact through one or, equally, several clothing layers 3, 7. Alternatively, clothing layers 3, 7 are provided with holes or cutout patterns, so that the electrode 2 and/or the electrically conductive surfaces 5, 6, 9, 10 can make contact electrically through the clothing layers 3, 7.

Undermost clothing layer 3 is provided with an electrode 2, in particular with a reference electrode, making contact with the skin surface of body 1 of the wearer of the clothing. Undermost clothing layer 3 is provided with an electrically conductive surface 5 on the side facing away from the skin surface. Clothing layer 7 lying above the latter and, optionally, further clothing layers are each provided on the underside and upper side with an electrically conductive surface 6, 9, which is preferably orientated in such a way that, when the clothing layers are worn, an at least partial overlapping and contacting of the conductive layers lying respectively directly opposite one another, for example 5 and 6 in the case of two clothing layers 3, 7, is ensured. In particular, the conductive surfaces are designed structured or in the form of hook and loop, eg Velcro, fasteners, so that the position of the individual clothing layers relative to one another and of the conductive surfaces is fixed.

A carrier belt 11 with a further conductive surface 10 turned towards the body side is then passed over the stack of at least two clothing layers 3, 7. A cable forming an electrical line 12 in carrier belt 11 is connected to an analysing unit 14, which is located for example in or on respiratory equipment 13. Analysing unit 14 communicates wireless in particular with an operational control room. Electrode 2 is bought into contact with the skin surface of body 1 by the mechanical contact pressure of carrier belt 11, which is brought about by the weight of respiratory equipment 13, and a good contact is produced between conductive surfaces 5, 6, 9, 10 of individual clothing layers 3, 7 up to carrier belt 11. A sufficiently good electrical contact is thus produced even under the severe conditions of a fire service deployment.

## Claims

1. An arrangement for the transmission of an electrical signal detected by contact with the skin surface to an analysing unit including:
a stack of at least two clothing layers (3,7) with an electrical line connection (4,8) through each of the clothing layers (3,7); in which
the undermost clothing layer (3) is provided with an electrode contactable with the skin surface of the body (1) of a wearer (2) of the clothing and with an electrically conductive surface (5) on the side facing away from the skin surface, in use; and
further clothing layer (7) stacked upon the undermost clothing layer (3) is provided on the underside and upper side with an electrically conductive surface (6,9); and in which
a carrier belt (11) passed over the stack of clothing layers (3,7) is provided with an electrically conductive surface (10) facing the uppermost clothing layer (7), said conductive surface being connected by means of an electrical line (12) to the analysing unit (14).

2. The arrangement according to claim 1, in which the carrier belt (11) is connected to respiratory equipment (13) on the back of the wearer of the clothing, so that the weight of the respiratory equipment (13) presses the carrier belt (11) onto the stack of clothing layers (3,7).

3. The arrangement according to claim 1 or 2, in which the undermost clothing layer (3) is provided with several electrodes, whereby one of the electrodes is a reference electrode for an ECG measurement.

4. The arrangement according to any one of the preceding claims, in which the electrically conductive surfaces (5,6,9,10) are a few square centimetres in size and are orientated in such a way that they overlap at least partially when the articles of clothing assigned to the clothing layers (3,7) are put on by the wearer of the clothing.

5. The arrangement according to any one of the preceding claims, in which the electrical line (12) is integrated into the carrier belt (11).

6. The arrangement according to any one of the preceding claims, in which the analysing unit (14) is connected wireless to an operational control room.

7. The arrangement according to any one of the preceding claims, in which the conductive surfaces (5,6,9,10) are glued, welded, sintered, woven, embroidered, woven or tufted.

8. The arrangement according to any one of the preceding claims, in which the conductive surfaces (5,6,9,10) are designed structured or in the form of hook and loop fasteners, so that the position of the individual clothing layers (3,7) and the carrier belt (11) with respect to one another and of the conductive surfaces (5,6,9,10) can be fixed.

9. The arrangement according to any one of the preceding claims, in which the electrical line connection (4,8), are electrically conductive threads, plug-in elements or push-contact elements which make contact respectively through one or more clothing layers (3,7).

10. The arrangement according to any one of claims 1 to 8, in which, for the electrical line connection (4,8) through the clothing layers (3,7), the latter are provided with holes or with cutout patterns, so that the electrode (2) and/or the electrically conductive surfaces (5,6,9,10) can make contact electrically through the clothing layers (3,7).
